# EUROPEAN PATENT APPLICATION

(11) **EP 4 813 322 A1**
(43) Date of publication of application: **30.09.2026**
(21) Application number: 25166132.8
(22) Date of filing: 25.03.2025
(51) Int. Cl.: A61B 17/32

(54) **ULTRASONIC CUTTING DEVICE**

(71) Applicant: Bosonic AG, 3010 Bern (CH)
(72) Inventor: BÜSCHER, Robin, Heikendorf (DE); VELTMEIJER, Ewoud, Bern (CH); BELAL, Rafik, Bern (CH)
(74) Representative: Frei Patent Attorneys

(57) **Abstract**

An ultrasonic cutting device (1) comprising a blade member (2) and an attachment member (3) for operatively connecting the ultrasonic cutting device (1) to a source of ultrasonic oscillation, wherein the blade member (2) and the attachment member (3) extend along a common longitudinal axis L, wherein the blade member (2) comprises a distal end (21), a proximal end (22) adjoining the attachment member (3), and a pair of oppositely facing lateral edges (23, 23'), wherein the blade member (2) comprises a distal cutting portion (24) adjoining the distal end (21) in a proximal direction and comprising a plurality of cutting teeth (26), and a proximal root portion (25) adjoining the cutting portion (24) in the proximal direction, and wherein the cutting teeth (26) become larger in a distal direction over substantially an entire axial length of the cutting portion (24).

## Description

### FIELD OF THE INVENTION

The invention resides in the field of medical technology and relates to a device for cutting into or through bone. It relates in particular to an ultrasonic cutting device suitable to be used with a source of ultrasonic mechanical vibrations.

### BACKGROUND OF THE INVENTION

In the field of surgery, it is often necessary to ablate or cut bone tissue, for example, for the correction of deformities in the foot and ankle area.

Various tools are known in the art for this purpose. For example, an osteotome, which is effectively a specialized form of chisel, can be forced longitudinally through a bone to be cut. If lateral cutting into bone is desired or required, bone saws can be used. While traditional osteotomes and saws are functional, they have many disadvantages. Depending on the force applied the directional accuracy and precision of the applied technique is compromised and surrounding structural bone is damaged, especially if it is weakened by osteoporosis or the like. In any case, manual sawing through bone is a tiring process that also leads to localized heating and copious bone swarf.

Another approach is to use powered devices, whether electric or pneumatic, of the reciprocating or rotary type. For example, US 2001/0037114 A1 discloses a surgical handpiece adapter for converting rotary motion of a powered surgical handpiece into reciprocating motion to drive a cutting member. However, such devices are quite complex and feature many moving parts, which makes them both expensive to manufacture and prone to failure. Because of the complicated mechanical mechanism for generating the reciprocating motion of the blade and the associated vibrations and shocks in the adapter, it is also difficult for the surgeon to move these devices in a precise fashion and without undesired damage of surrounding healthy tissue. Moreover, it is generally not easy for reciprocating saws to initiate and direct a cut because a cut must start from an edge or, alternatively, a starting hole which needs to be created first by a drill or similar instrument.

Recent years have seen increased usage of minimally invasive surgical procedures (MIS). These minimally invasive surgical procedures have the advantage of involving smaller incisions and placing less of a burden upon the patient's body than conventional surgical procedures in which large incisions are made. Due to the small surgical device insertion opening (portal) in minimally invasive surgical procedures, in the order of few millimeters, thick and/or wide blades cannot be used. Instead, rotatable surgical burrs, such as disclosed in EP 0 836 833 A2, having a rotatable cutting element at the distal end of a bendable non-rotating elongated outer member are used. However, when a rotary blade is used, the cut must follow a relatively straight path to prevent the blade from binding in the cut. Furthermore, such burrs may also be difficult to guide accurately, and flexure in their elongate rotating drive shafts may lead to unacceptable collateral damage in surrounding structural bone and soft tissue. Moreover, they tend to produce inconveniently large quantities of bone swarf, which must be removed to allow clear visualization of the point at which the burr is being used. Since burrs rotate at high speeds to work effectively and efficiently, they lead to significant localized frictional heating, which may also harm adjacent bone, tissue or marrow.

Ultrasonic cutting devices as disclosed, for example, in US 5,261,922 overcome many of the disadvantages and problems discussed above. An ultrasonic cutting device commonly comprises an elongated tip or tool or knife blade connected to an ultrasonic oscillation source. The edge of the knife blade is brought into direct contact with the tissue being operated on and vibrated at ultrasonic frequencies. The use of ultrasonic cutting devices is preferred over that of traditional, manual or motorized cutting devices, because the ultrasonic vibrations favor the penetration of the blade reducing the force required by a surgeon, which allows for greater precision in making a cut.

Nevertheless, room for improvement remains. In particular, with the known ultrasonic cutting blades, the ability to create thin and precise curved cuts is extremely limited because the known blades are prone to break in the process, especially when attempting lateral and/or low-radius cuts. Furthermore, the known blades are not well suited for lateral, i.e. side cutting because their blade thickness, i.e. cutting kerf, and their blade width prevents them from cutting easily through the tissue to be cut. On the other hand, thin blades do not withstand the bending stresses that occur during side cutting and they also tend to break due to a lack of mechanical stability. This significantly limits or even precludes their applicability in minimally invasive surgical procedures.

It is therefore an object of the invention to remedy at least one of the afore-mentioned disadvantages of the prior art and in particular to provide an ultrasonic cutting device, in particular for use in minimally invasive surgery, that allows for improved side-cutting and/or curved cutting of bone, and that is gentle on soft tissue surrounding the bone.

### SUMMARY OF THE INVENTION

The object is achieved by an ultrasonic cutting device as defined in the patent claims.

The object is in particular achieved by an ultrasonic cutting device comprising a blade member and an attachment member for operatively connecting the ultrasonic cutting device to a source of ultrasonic oscillation. The blade member and the attachment member extend along a common longitudinal axis L. The blade member comprises a distal end, a proximal end adjoining the attachment member, and a pair of oppositely facing lateral edges. The blade member further comprises a distal cutting portion adjoining the distal end in a proximal direction and comprising a plurality of cutting teeth. The blade member further comprises a proximal root portion adjoining the cutting portion in the proximal direction. The cutting teeth become larger in a distal direction over substantially an entire axial length of the cutting portion. In other words, the cutting teeth in an area of the cutting portion opposite the distal end are smaller than the cutting teeth in an area adjacent the distal end, with the cutting teeth arranged in between having an intermediate size.

The inventors have surprisingly found that the problem of breaking due to bending stresses, which occurs with the thin blades known from the prior art, can be overcome or at least significantly reduced by making the size of the cutting teeth larger in the direction of the distal end. By enlarging the cutting teeth in the distal direction and, correspondingly, by making larger recesses in the lateral edges from which the cutting teeth are machined, more aggressive cutting teeth are provided in the distal direction on the one hand, and on the other hand the mechanical stability of the blade in the proximal end, or at the transition from the blade member to the attachment member, are achieved. It follows that the ultrasonic cutting device disclosed herein is characterized by a favorable bending strength, which can withstand bending forces occurring during lateral cutting, i.e. side cutting. As a result, the ultrasonic cutting device allows for highly effective side cutting, in particular in a curved motion. In addition, the weight savings achieved by making larger recesses between the cutting teeth towards the distal end of the blade member helps with ultrasonic tuning and hitting the right frequency.

The attachment member of the ultrasonic cutting device disclosed herein may comprise any means by which the ultrasonic cutting device may detachably be connected to a generator of ultrasonic mechanical vibratory energy, particularly to a stack of piezoelectric crystal elements and a waveform generator that applies an ultrasonic-frequency voltage across the piezo-stack. For example, the attachment member may comprise a bayonet lock or an externally or internally threaded connector for operatively linking the ultrasonic cutting device and its blade member to a source of ultrasonic oscillation.

Generally, all terms used in the present disclosure are to be interpreted according to their ordinary meaning in the technical field, unless explicitly defined otherwise herein.

When the term "distal direction" is used, this refers to the direction pointing away from the attachment member or towards the distal end, respectively. When the term "distal end" is used, this refers to the end of the ultrasonic cutting device which during use of the ultrasonic cutting device is free and located furthest away from the attachment member. Correspondingly, when the term "proximal direction" is used, this refers to the direction pointing towards the attachment member.

In particular, the cutting teeth become progressively larger in a distal direction over substantially an entire axial length of the cutting portion.

The terms "progressive" and "progressively" are understood to mean a continuous change in a value, for example a thickness of the blade member, a tooth height and/or a tooth width. In particular, the terms "progressive" and "progressively" do not refer to a step-like change in value, i.e. a constant and then a more or less abrupt change in a value such as a thickness of the blade member, a tooth height and/or a tooth width.

In particular, the proximal end of the blade member is defined as the point along the longitudinal axis L at which the thickness of the blade member exceeds 3 mm.

Further, the terms "longitudinal", "longitudinally", "axially" and "axial" refer to a direction extending from a connecting end of the attachment member to the distal end of the blade member and along the ultrasonic cutting device.

Similarly, the terms "radial", "radially", "transverse", "transversal" and "transversally" refer to a direction generally perpendicular to the longitudinal axis L.

In embodiments the cutting teeth each have a tooth height h defined between a base and a tip of the respective tooth. The tooth height h of the cutting teeth becomes larger in the distal direction over substantially the entire axial length of the cutting portion. In particular, the tooth height h of the cutting teeth becomes progressively larger in the distal direction over substantially the entire axial length of the cutting portion.

The term "over substantially the entire length of the cutting portion" means in particular that a few cutting teeth, starting from the distal end, may first increase in size before the size of the remaining cutting teeth arranged at the respective lateral edge progressively decreases in the proximal direction. In particular, the first two cutting teeth on each lateral edge may increase in size before the size of the remaining cutting teeth decreases in the proximal direction, in particular progressively. This is due, for example, to the fact that the distal end is tapered.

In embodiments the tooth height h is between 0.1 and 1.2 mm. The inventors have found that cutting teeth of this size are particularly suitable with regard to an overall width of the blade, which should not be exceeded in order to facilitate or improve a curved cutting ability of the ultrasonic cutting device, as will be explained in more detail further below.

In embodiments a tooth gap is formed between the tips of every two adjacent cutting teeth, wherein the tooth gaps increase in the distal direction. This reduces the oscillating mass of the blade member towards the distal end, which has a favorable effect on the load on the blade. It also increases the cutting performance or aggressiveness of the cutting teeth in the distal direction while retaining the stability at the proximal end where the highest bending loads are expected to occur.

In embodiments the cutting teeth each have a tooth width w defined at their widest point, measured in a direction of the longitudinal axis L. The tooth width w of the plurality of cutting teeth is arranged to decrease, in particular gradually, in a direction away from the distal end over substantially the entire axial length of the cutting portion.

The term "over substantially the entire length of the cutting portion" means in particular that the tooth width of a few cutting teeth, starting from the distal end, may first increase before the tooth width of the remaining cutting teeth arranged at the respective lateral edge decreases in the proximal direction, in particular progressively. In particular, the first two cutting teeth on each lateral edge may feature an increasing tooth width before the tooth width of the remaining cutting teeth decreases in the proximal direction, in particular progressively.

In embodiments a ratio of the tooth height h to the tooth width w ranges from 0.2 to 2. Preferably, the ratio of the tooth height h to the tooth width w ranges from 0.5 to 1.5. In this range, a particularly favorable balance is achieved between the mechanical stability of the teeth and their cutting performance.

In embodiments each tooth of the plurality of cutting teeth has at least two facets with a generally triangular shape. Such cutting teeth can be easily machined form the solid by making lateral cuts and beveling the top and bottom surfaces of the blade member.

In embodiments the lateral edges each run through the tips of the cutting teeth arranged on the respective lateral edge. This type of blade shape is very suitable for producing straight lateral cuts and can also be produced particularly efficiently from rectangular blade member blanks.

In embodiments the distal end and the proximal end define a working length 1 along the longitudinal axis L. The working length l is between 15 mm and 30 mm. Preferably, the working length is between 20 mm and 25 mm. In particular, the working length is about 24 mm.

In embodiments the cutting teeth are arranged over 50% to 80% of the working length l. Preferably, the cutting teeth are arranged over 70% to 80% of the working length l. This means that the blade member has areas or sections without teeth that protect the tissue or do not injure it.

In embodiments the lateral edges in the root portion are free of cutting teeth. As a result, the blade member has a portion that is gentle to soft tissue surrounding the hard tissue, i.e. bone to be cut or does not damage the soft tissue after the cutting portion of the ultrasonic surgical instrument has been inserted into the bone.

In embodiments the oppositely facing lateral edges are parallel to one another over at least 80% of the cutting portion. Preferably, the oppositely facing lateral edges are parallel to one another over at least 90% of the cutting portion. Such a shape enables particularly straight cuts and particularly efficient production. The distal end may, for example, have a tapered shape. In particular, the distal end may have a pointed shape which makes it easier to insert the cutting portion of the ultrasonic cutting device into the portal or to make holes in the bone.

The root portion may in principle have any cross-section such as a substantially cylindrical, rectangular, or diamond-shaped cross-section. In embodiments the root portion is hollow. This reduces the mass in the area of the distal end, which has a beneficial effect on the bending strength of the blade member.

In embodiments the cutting teeth are oriented generally transversely to the longitudinal axis L. This ensures that the cutting portion has the same cutting performance both when moving forwards and backwards, i.e. both in the distal direction and in the proximal direction.

In embodiments the cutting teeth are arranged symmetrically in relation to the longitudinal axis L. In particular, the cutting teeth are arranged mirror-symmetrically to the longitudinal axis L along the longitudinal axis L. A symmetrical design ensures uniform distribution of vibrations, reducing energy loss and improving efficiency. It also minimizes lateral forces, i.e. it ensures that forces remain directed along the intended axis. This way, mechanical stresses are distributed more evenly, which reduces the risk of material fatigue and increases the longevity of the instrument. Symmetry allows for more uniform energy distribution across the cutting surface, leading to better control and more consistent surgical outcomes by reducing unintended or asymmetrical loading.

In embodiments the blade member has a plurality of chamfers, in particular four chamfers. In particular the cutting portion of the blade member has a plurality of chamfers, in particular four chamfers. Without such chamfers, the teeth would have a substantially rectangular cross-section, and the outer corners thereof may be prone to damage upon contact with hard tissue or prosthesis. It follows that there would be a significant chance of these corners being knocked off, notched or chipped as a result. Such loss of material from the teeth would lead to an imbalance of the blade and could result in the blade having to be disposed of. By contrast, a chamfered blade member avoids such problems to a great extent as the teeth are set back from at least one of a top and bottom of the blade member, and are less likely to be damaged. Moreover, the chamfers reduce the cutting resistance when cutting laterally, thereby improving the cutting performance of the ultrasonic surgical device.

In embodiments the attachment member comprises a central channel and at least one irrigation aperture in fluid communication with the central channel. The at least one irrigation aperture is arranged adjacent the proximal end of the blade member and configured for dispensing an irrigation fluid onto the blade member, in particular onto its root portion. Preferably, the attachment member comprises a central channel and two irrigation apertures in fluid communication with the central channel. This can be used to cool the blade and/or rinse the surgical site. Two irrigation apertures arranged symmetrically, in particular mirror-symmetrically in relation to the longitudinal axis L bring about the advantages described above with regard to the symmetrical arrangement of the cutting teeth.

In embodiments a thickness d of the blade member tapers continuously from the proximal end towards the distal end. The thickness d of the blade member is defined by a distance between a top surface of the blade member and a bottom surface of the blade member opposite the top surface, measured perpendicular to a plane extending through the plurality of cutting teeth.

In the context of the present invention, the term "continuous taper" means an uninterrupted, smooth decrease without any pauses or sudden jumps. Accordingly, "tapers continuously" means that at every instant along the longitudinal axis the cross-section is reducing towards the distal end, without discrete steps.

A tapered design reduces the cross-sectional area of the blade member gradually towards the distal end, allowing a smoother transition of stress along the length of the blade member, which helps to prevent stress concentration that could lead to mechanical failure. Also, a tapered design minimizes abrupt changes in stiffness and leads to lower internal friction and heat generation, reducing the likelihood of localized stress accumulation that can cause material fatigue over time. Also, a tapered design allows for some flexibility at the distal end while strengthening the proximal end and maintaining structural integrity at the proximal end where forces on the blade member are highest during lateral cutting. This way, the lifetime of the ultrasonic surgical device can be prolonged.

The thickness d of the blade member may taper evenly from its proximal end to its distal end, i.e. the thickness d decreases linearly in the distal direction. Alternatively, the thickness d of the blade may also change degressively from the proximal end to the distal end, i.e. the thickness d initially decreases more strongly in the direction of the distal end and then increasingly less strongly. It is also conceivable that the thickness of the blade member decreases in a combination of degressive and linear tapering towards the distal end.

In embodiments the blade member has a distal thickness d₂₁ of between 0.5 mm and 0.7 mm at its distal end and a proximal thickness d₂₂ of at most 3 mm at its proximal end. In these thickness ranges, a particularly advantageous balance is achieved between the mechanical load-bearing capacity and the cutting behavior of the blade member.

In embodiments the oppositely facing lateral edges define a width b of the blade member transverse to the longitudinal axis L. In particular, the tips of two cutting teeth arranged opposite each other on the lateral edges define a width b of the blade member between them. The width b of the blade member is between 2 mm and 4 mm, preferably between 2.5 mm and 3.5 mm, in particular about 3 mm. Again, in these width ranges, a particularly advantageous balance is achieved between the mechanical load-bearing capacity and the cutting behavior of the blade member.

In embodiments the cutting portion has a cross-sectional profile adjacent the distal end tapering towards the distal end in both width b and thickness d of the blade member.

In embodiments the distal end is pointed with a substantially pyramidal tip shape. Such a cross-sectional profile adjacent the distal end provides better access to confined areas and allows for more precise incisions, minimizing collateral damage to surrounding tissues.

In embodiments a sum of the tooth heights h of cutting teeth arranged opposite one another in the region of the distal end, relative to the blade member width b, is at least 40%, preferably at least 45%, in particular about 50%. Such a design is characterized by particularly aggressive cutting teeth towards the distal end of the blade member and thus a particularly good cutting performance.

Ther term "in the region of the distal end" means in particular the region along the longitudinal axis L of the blade member that extends no further than four pairs of oppositely arranged teeth in the direction of the proximal end from the distal end.

### BRIEF DESCRIPTION OF THE DRAWINGS

Embodiments of the present disclosure will now be described by way of example only and with reference to the following accompanying figures in which the same or corresponding elements are generally labelled with the same reference signs. The figures show:
- Figure 1a: shows a schematic drawing of an exemplary embodiment of an ultrasonic cutting device in a perspective view;
- Figure 1b: shows the ultrasonic cutting device of Figure 1a in a top view;
- Figure 1c: shows the ultrasonic cutting device of Figure 1a in a side view;
- Figure 2a: shows a schematic drawing of another exemplary embodiment of an ultrasonic cutting device in a perspective view;
- Figure 2b: shows the ultrasonic cutting device of Figure 2a in a top view;
- Figure 2c: shows the ultrasonic cutting device of Figure 2a in a side view;
- Figure 2d: shows a longitudinal section through the side view of Figure 2c along the longitudinal axis L;
- Figure 2e: shows a magnification of the longitudinal section shown in Figure 2d in the area of the blade member 2;
- Figure 2f: shows a longitudinal section through the top view of Figure 2b along the longitudinal axis L;
- Figure 2g: shows a magnification of the longitudinal section shown in Figure 2f in the area of the cutting portion 24;
- Figure 3a: shows a schematic drawing of another exemplary embodiment of an ultrasonic cutting device in a perspective view;
- Figure 3b: shows the ultrasonic cutting device of Figure 3a in a top view;
- Figure 3c: shows the ultrasonic cutting device of Figure 3a in a side view;
- Figure 4a: shows a schematic drawing of another exemplary embodiment of an ultrasonic cutting device in a perspective view;
- Figure 4b: shows the ultrasonic cutting device of Figure 4a in a top view;
- Figure 4c: shows the ultrasonic cutting device of Figure 4a in a side view;
- Figure 5a: shows a schematic drawing of another exemplary embodiment of an ultrasonic cutting device in a perspective view;
- Figure 5b: shows the ultrasonic cutting device of Figure 5a in a top view;
- Figure 5c: shows the ultrasonic cutting device of Figure 5a in a side view;
- Figure 6a: shows a schematic drawing of another exemplary embodiment of an ultrasonic cutting device in a perspective view;
- Figure 6b: shows the ultrasonic cutting device of Figure 6a in a top view;
- Figure 6c: shows the ultrasonic cutting device of Figure 6a in a side view;
- Figure 7a: shows a schematic drawing of another exemplary embodiment of an ultrasonic cutting device in a perspective view;
- Figure 7b: shows the ultrasonic cutting device of Figure 7a in a top view;
- Figure 7c: shows the ultrasonic cutting device of Figure 7a in a side view.

### DESCRIPTION OF THE PREFERRED EMBODIMENTS

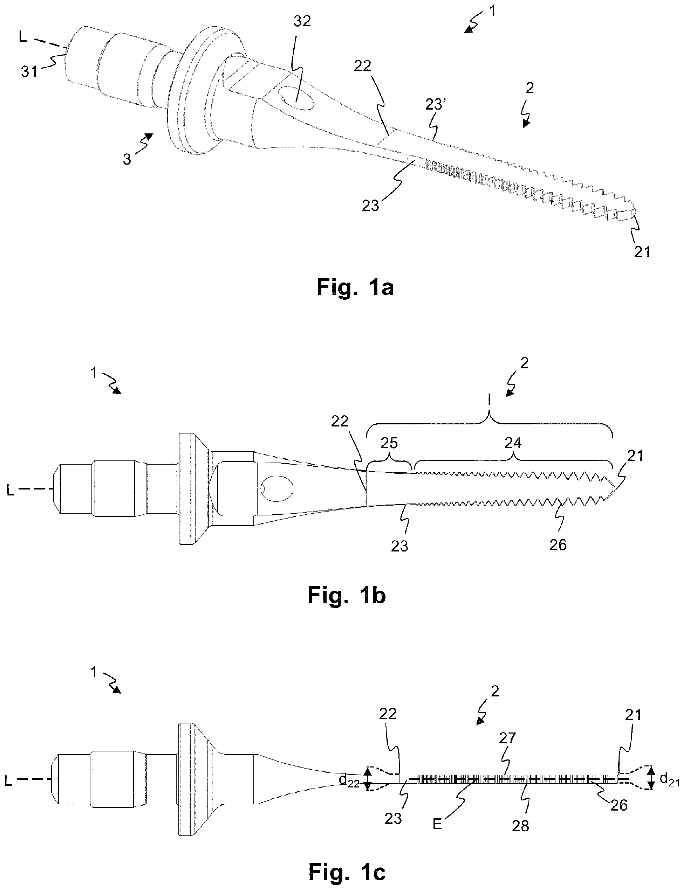

Figure 1a shows an exemplary embodiment of an ultrasonic cutting device 1 in a perspective view. The ultrasonic cutting device 1 comprises a blade member 2 and an attachment member 3 for operatively connecting the ultrasonic cutting device 1 to a source of ultrasonic oscillation. The attachment member 3 can be designed, for example, as a thread or as part of a bayonet lock. Both the attachment member and the blade member 2 extend along a common longitudinal axis L, which is indicated in Figure 1a by a dashed line at an attachment end of the ultrasonic cutting device 1. The blade member 2 comprises a distal end 21, a proximal end 22 adjoining the attachment member 3, and a pair of oppositely facing lateral edges 23, 23'. As will be described in more detail in the following embodiments, the attachment member 3 of the embodiment shown in Figures 1a-1c comprises a central channel 31 and two irrigation apertures 32 in fluid communication with the central channel 31, whereby only one of the two irrigation apertures 32 is visible in the representation of Figure 1a and the second irrigation aperture is arranged mirror-symmetrically to the first irrigation aperture 32. Both the first and the second irrigation aperture 32 are arranged adjacent the proximal end 22 of the blade member 2 and configured for dispensing an irrigation fluid onto the blade member 2.

Figure 1b shows the ultrasonic cutting device 1 of Figure 1a in a top view. As can be seen from this view, the distal end 21 and the proximal end 22 define a working length l along the longitudinal axis L, wherein the working length l of the blade member 2 consists of a distal cutting portion 24 adjoining the distal end 21 in a proximal direction, and a proximal root portion 25 adjoining the cutting portion 24 in the proximal direction to the proximal end 22. The cutting portion 24 is characterized by the presence of a plurality of cutting teeth 26. The cutting teeth 26 become larger in a distal direction over substantially an entire axial length of the cutting portion 24. This is demonstrated in Figure 1b by the fact that of the twenty-seven cutting teeth 26 arranged on each of the two oppositely facing lateral edges 23, only the two cutting teeth closest to the distal end 21 are no longer larger than their respective neighboring cutting tooth in the proximal direction.

Figure 1c shows the ultrasonic cutting device 1 of Figure 1a in a side view. At the distal end 21, the blade member 2 features a thickness d₂₁ defined by a distance between a top surface 27 of the blade member 2 and a bottom surface 28 of the blade member 2 opposite the top surface 27, measured perpendicular to a plane E extending through the plurality of cutting teeth 26, of about 3 mm. At the proximal end 22, the blade member 2 features a thickness d₂₂, which in the embodiment shown in Figures 1a-1c is substantially identical to the thickness d₂₁ at the distal end 21. Put differently, the blade member 2 has a constant thickness d over essentially its entire axial length. The lateral edges 23 in the root portion are free of cutting teeth 26.

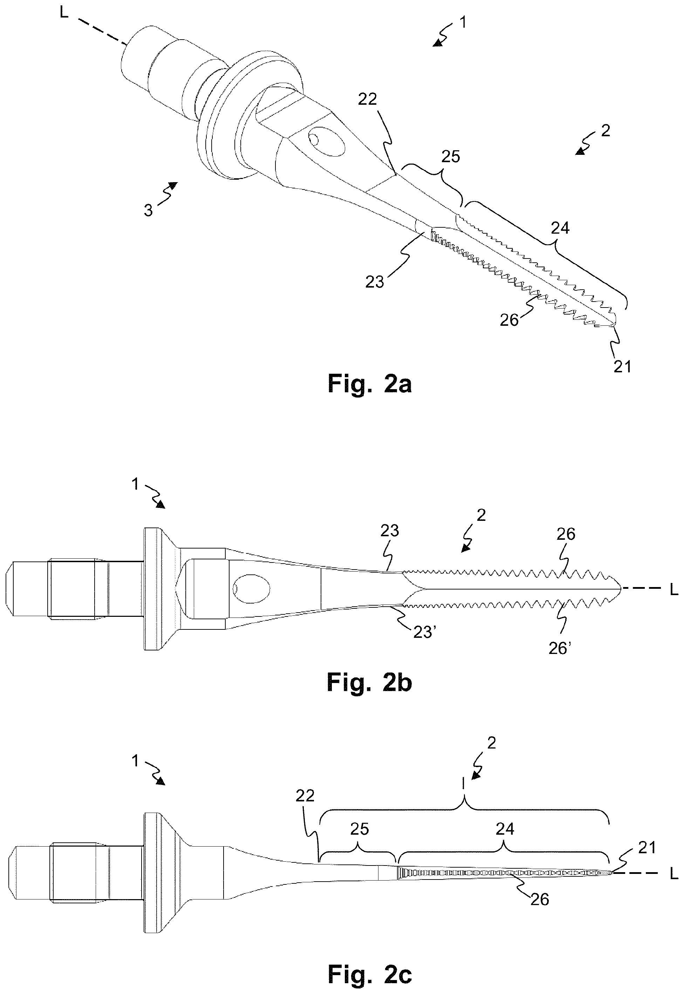

Figure 2a shows another exemplary embodiment of an ultrasonic cutting device 1 in a perspective view. The ultrasonic cutting device 1 comprises a blade member 2 with a free distal end 21, a pair of oppositely facing lateral edges, of which, in the perspective view shown in Figure 2a, only the lateral edge facing the observer is provided with the reference sign 23, and a proximal end 22 adjoining an attachment member 3 for operatively connecting the ultrasonic cutting device 1 to a source of ultrasonic oscillation. The attachment member 3 can, for example, comprise a threaded connection which can be screwed into a corresponding counter-tread in a handpiece for the ultrasonic cutting device 1. The blade member 2 comprises a distal cutting portion 24 adjoining the distal end 21 in a proximal direction, and a proximal root portion 25 adjoining the cutting portion 24 in the proximal direction. The blade member 2 and the attachment member 3 extend along a common longitudinal axis L, which is indicated by the dashed line in Figure 2a. The cutting portion 24 comprises a plurality of cutting teeth 26 that become progressively larger in a distal direction, i.e. towards the distal end 21, over substantially an entire axial length of the cutting portion 24. Put differently, the cutting portion 24 is defined along the longitudinal axis L by the presence of cutting teeth 26, with the cutting teeth 26 progressively decreasing in size from the distal end 21 toward the proximal end 22. In the embodiment shown in Figure 2a, a cross-section of the blade member 2 progressively tapers toward the distal end, with the blade member 2 in the root portion 25 having a substantially rectangular cross-section. The cutting portion 24 has four chamfers, i.e. four beveled edges, so that a cross-section at the boundary between two neighboring cutting teeth 26 has a substantially hexagonal cross-section. Accordingly, in the embodiment of the ultrasonic cutting device 1 shown in Figures 2a-2g, each tooth 26 of the plurality of cutting teeth 26 has two facets with a generally triangular shape. Starting from the distal end 21, the cutting portion 24 has cross-sections running through the tips of the cutting teeth 26, which change from a substantially diamond-shaped cross-section to a hexagonal cross-section in the direction of the proximal end 22.

Figure 2b shows the ultrasonic cutting device 1 of Figure 2a in a top view. As can be seen from this view, the cutting teeth 26, 26' of blade member 2 are oriented generally transversely to the longitudinal axis L. Moreover, the cutting teeth 26, 26' are arranged symmetrically in relation to the longitudinal axis L so that the teeth 26, 26' arranged on oppositely facing lateral edges 23, 23' each point in opposite directions in pairs.

Figure 2c shows the ultrasonic cutting device 1 of Figure 2a in a side view. The blade member 2 has a working length l of about 24 mm, measured along the longitudinal axis between the distal end 21 and the proximal end 22. In other words, the distal end 21 and the proximal end 22 define the working length l along the longitudinal axis L. Accordingly, the working length l is the sum of the axial lengths of the root portion 25 and the cutting portion 24. As can be seen from Figure 2c, the cutting teeth 26 become progressively smaller in size in the proximal direction of the ultrasonic cutting tool 1 and cease to be present in the root portion 25, i.e. the root portion 25 is free of cutting teeth 26. In the embodiment shown in Figures 2a-2g, the cutting teeth 26 are arranged over about 75% of the working length l.

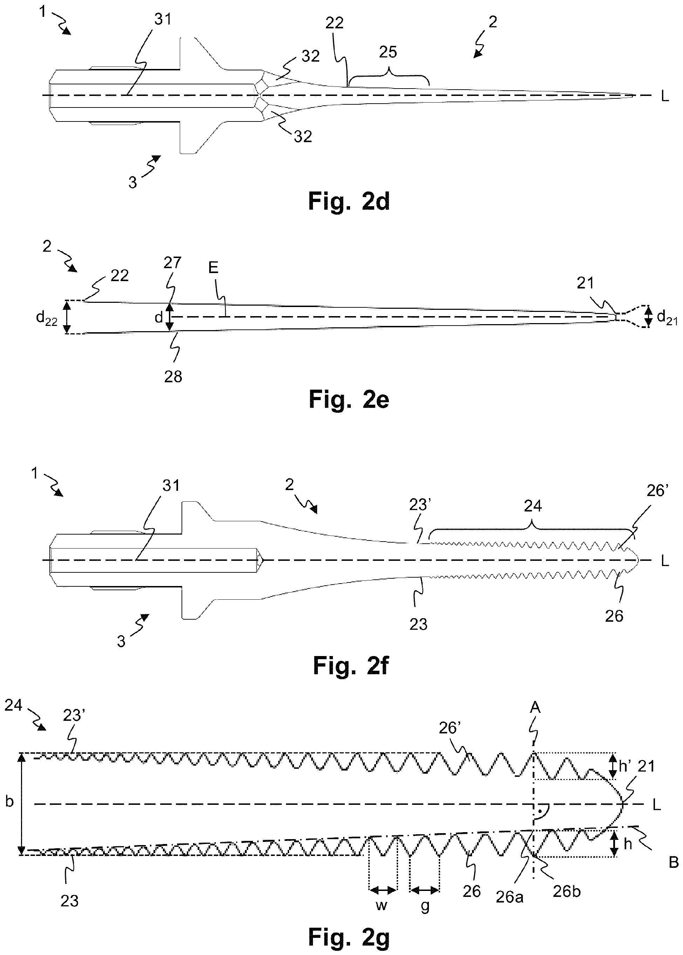

Figure 2d shows a longitudinal section through the side view of Figure 2c along the longitudinal axis L. As can be seen from this section, the attachment member 3 of the ultrasonic cutting device 1 comprises a central channel 31, i.e. a bore extending from a distal end of the attachment member 3 along the longitudinal axis L and over a part of an axial length of the attachment member 3, as well as two irrigation apertures 32.

The two irrigation apertures 32 are in fluid communication with the central channel 31 and arranged adjacent the proximal end 22 of the blade member 2 so that an irrigation fluid such as an aqueous solution can run from a handpiece not shown in Figure 2d via the central channel 31 and the two irrigation apertures 32 and onto the root portion 25 of blade member 2.

Figure 2e shows a magnification of the longitudinal section shown in Figure 2d in the area of the blade member 2. A thickness d of the blade member 2 tapers continuously from the proximal end 22 towards the distal end 21. The thickness d of the blade member 2 is defined by a distance between a top surface 27 of the blade member 2 and a bottom surface 28 of the blade member 2 opposite the top surface 27, measured perpendicular to a plane E extending through the plurality of cutting teeth, which in this plane of projection extend backwards and are therefore not visible in Figure 2e.

Accordingly, the blade member 2 comprises a distal thickness d₂₁ of about 0.6 at the distal end 21 and a proximal thickness d₂₂ of about 3 mm at the proximal end 22.

Figure 2f shows a longitudinal section through the ultrasonic cutting device 1 shown in a top view in Figure 2b along the longitudinal axis L. The central channel 31 can be seen, which extends symmetrically around the longitudinal axis L and over part of the attachment member 3. The blade member 2 has a first lateral edge 23 and a second lateral edge 23' arranged opposite the first lateral edge 23, which run mirror-symmetrically to the longitudinal axis L. Both lateral edges 23, 23' each have a plurality of cutting teeth 26, 26' on a section of the respective lateral edges 23, 23'. The presence of cutting teeth 26, 26' defines the cutting portion 24 of the blade member 2. In accordance with the symmetrical design of the ultrasonic cutting device 1, the cutting teeth 26, 26' are also arranged in a mirror-symmetrical manner with respect to the longitudinal axis L, i.e. the cutting teeth 26, 26' are aligned with each other along respective directions that are transverse relative to the longitudinal axis L.

Figure 2g shows a magnification of the longitudinal section shown in Figure 2f in the area of the cutting portion 24. The blade member has a width b of about 3 mm defined by a distance between the oppositely facing lateral edges 23, 23' transverse to the longitudinal axis L or the distance between the tips of oppositely facing cutting teeth 26, 26', respectively. The cutting teeth 26, 26' each have a tooth heigh h, h' of between 0,1 mm and 1.2 mm, defined between a base 26a and a tip 26b of the respective tooth 26, 26'. The base of each cutting tooth 26, 26' is defined by the intersection of a first auxiliary line A and second auxiliary line B, which are each drawn with dots and dashes, respectively. The first auxiliary line A runs through the tip 26b of the tooth in question perpendicular to the longitudinal axis L. The second auxiliary line B runs through the valley bottoms located between the cutting teeth arranged on the lateral edge 23. The tooth heigh h of the cutting teeth 26, 26' becomes progressively larger in the distal direction, i.e. towards the distal end 21, over substantially the entire axial length of the cutting portion 24. The lateral edges 23, 23' each run through the tips 26b of the cutting teeth 26, 26' arranged on the respective lateral edge 23, 23', as indicated by the dashed lines with reference signs 23, 23'. The distance between the tips 26b of two cutting teeth 26, 26' arranged adjacent to each other on a respective lateral edge 23, 23' defines a tooth gap g, which increases in the distal direction. Moreover, the cutting teeth 26, 26' each have a tooth width w defined at their widest point, measured in the direction of the longitudinal axis L. In Figure 2g, the respective tooth widths can be inferred from the distance between the neighboring valley bottoms of the respective cutting tooth along the second auxiliary line B. The tooth width w of the respective cutting teeth 26, 26' gradually decreases in a direction away from the distal end 21 over substantially the entire axial length of the cutting portion 24. In the embodiment shown in Figures 2a-2g, the height h and tooth width of the cutting teeth 26, 26' become progressively larger in the distal direction over substantially the entire axial length of the cutting portion 24 as only four of the fifty-four cutting teeth 26, 26', namely the four cutting teeth closest to the distal end 21, do not increase in height h and tooth width w in the distal direction.

In the embodiment shown in Figures 2a-2g, the root portion 25 is not hollow. However, for reasons of weight reduction, it is also conceivable that the root portion 25 is hollow.

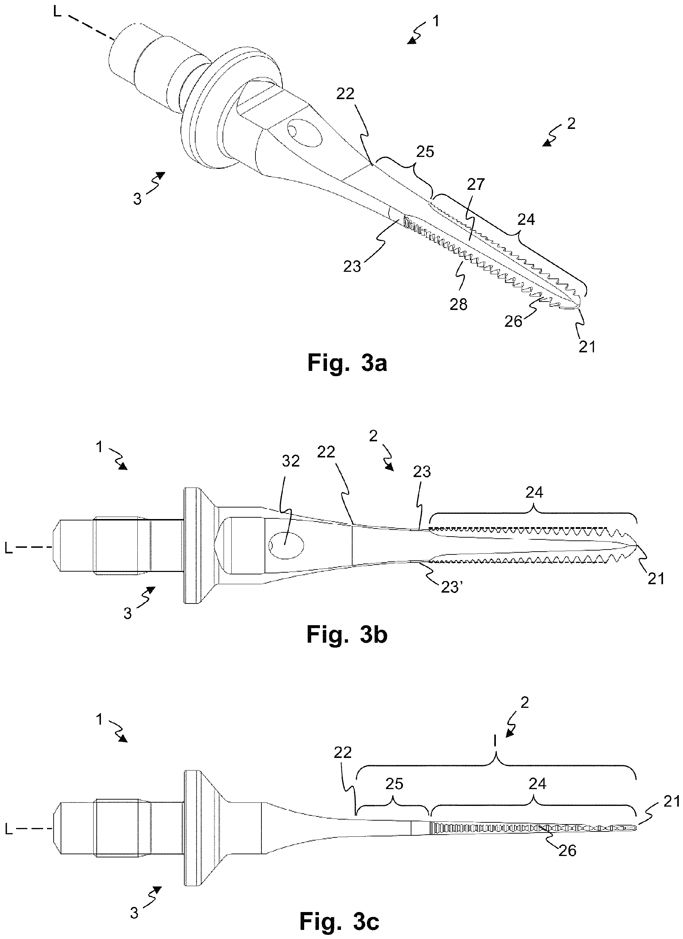

With regard to the embodiments shown in Figures 3a-3c, 4a-4c, 5a-5c, 6a-6c and 7a-7c, only selected features will be described in more detail below. For any remaining reference signs contained in these figures, reference is made to the description for Figures 2a-2g, which - with the exception of specific dimensions - applies analogously to the other figures.

Figure 3a shows a schematic drawing of another exemplary embodiment of an ultrasonic cutting device 1 in a perspective view. A cross-section of the blade member 2 progressively tapers toward the distal end, with the blade member 2 in the root portion 25 having a substantially rectangular cross-section. The cutting portion 24 has four chamfers, i.e. four beveled edges, whereby the chamfers - unlike in the embodiment of the ultrasonic cutting device 1 shown in Figures 2a-2g - do not adjoin on one another on the top surface 27 and the bottom surface 28 of the blade member 2. Thus, a cross-section at the boundary between two neighboring cutting teeth 26 has a substantially octagonal cross-section. Accordingly, in the embodiment of the ultrasonic cutting device 1 shown in Figures 3a-3c, each tooth 26 of the plurality of cutting teeth 26 has two facets with a generally triangular shape. Starting from the distal end 21, the cutting portion 24 has cross-sections running through the tips of the cutting teeth 26, which change from a hexagonal cross-section to an octagonal cross-section in the direction of the proximal end 22. The oppositely facing lateral cutting edges 23, 23' are parallel to one another over about 86% of the cutting portion 24, as indicated by the dashed lines in Figure 3b indicating the lateral edges 23, 23' extending through the tips of the cutting teeth 26 arranged on the respective lateral edges 23, 23'. As can be seen from the top and side views of the ultrasonic cutting device 1 in Figures 3b and 3c, respectively, the distal end 21 is sharpened, with the cutting portion 24 adjacent the distal end 21 tapering towards the distal end 21 in both the width and thickness of the blade member 2.

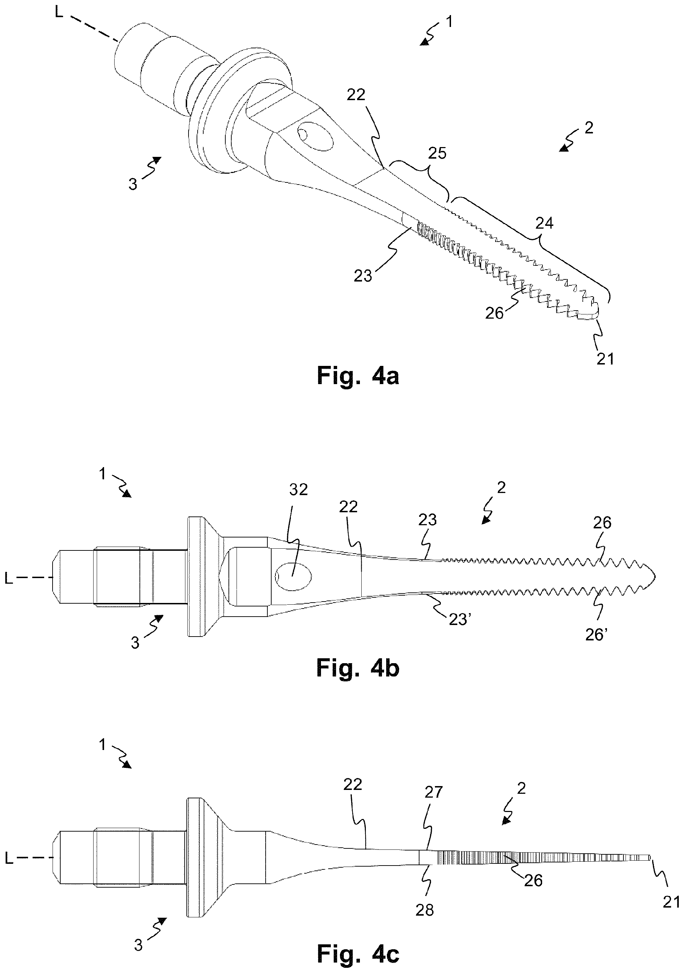

Figure 4a shows a schematic drawing of another exemplary embodiment of an ultrasonic cutting device 1 in a perspective view. As can be seen in particular from the side view in Figure 4c, the distance between the top surface 27 and the bottom surface 28 of the blade member 2, i.e. the thickness of the blade member 2, is constantly decreasing towards the distal end 21. Unlike the embodiments shown in Figures 2a-2g and 3a-3c, the blade member 2 of the embodiment shown in Figures 4a-4c does not feature chamfered lateral edges 23, 23'. Accordingly, blade member 2 has a substantially rectangular cross-section along the longitudinal axis L.

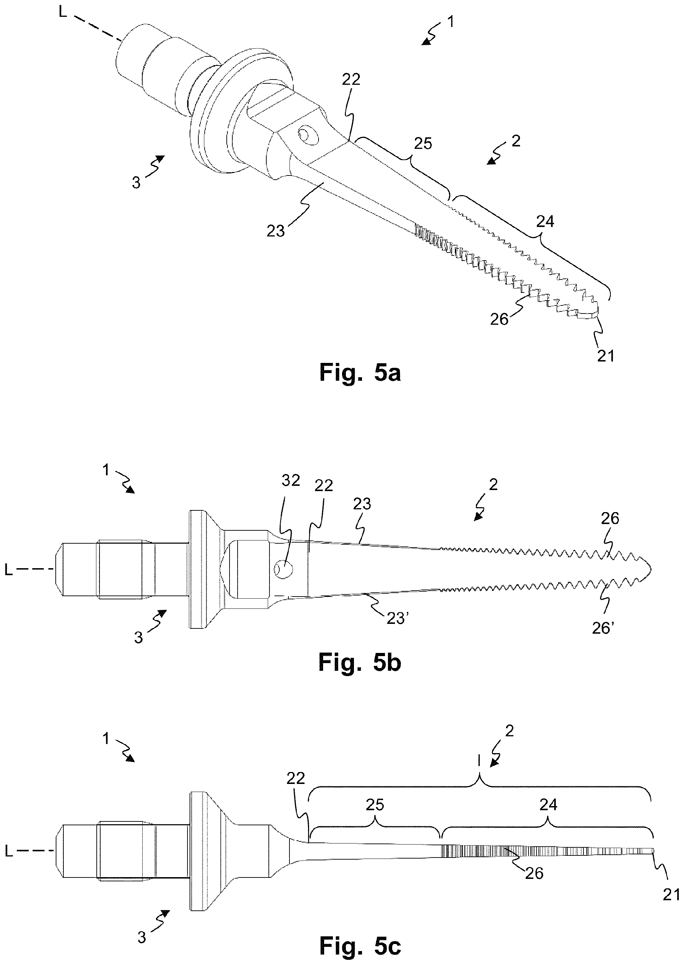

Figure 5a shows a schematic drawing of another exemplary embodiment of an ultrasonic cutting device 1 in a perspective view. The embodiment shown in Figures 5a-5c does not feature chamfered lateral edges 23, 23'. Accordingly, blade member 2 has a substantially rectangular cross-section along the longitudinal axis L. Compared with the embodiments shown and described in Figures 2a-2g, 3a-c, and 4a-c, the root portion 25 of the blade member 2 takes up a larger proportion of the entire working length 1, which is composed of the root portion 25 and the cutting portion 24. In other words, the root portion 25 is longer than in the embodiments described above. Since the lateral edges 23, 23' are not sharpened in the area of the root portion 25, the danger of inflicting unwanted damage in use of the ultrasonic cutting device 1 is reduced, in particular if the bone to be cut is surrounded by a thicker layer of soft tissue that should not be injured.

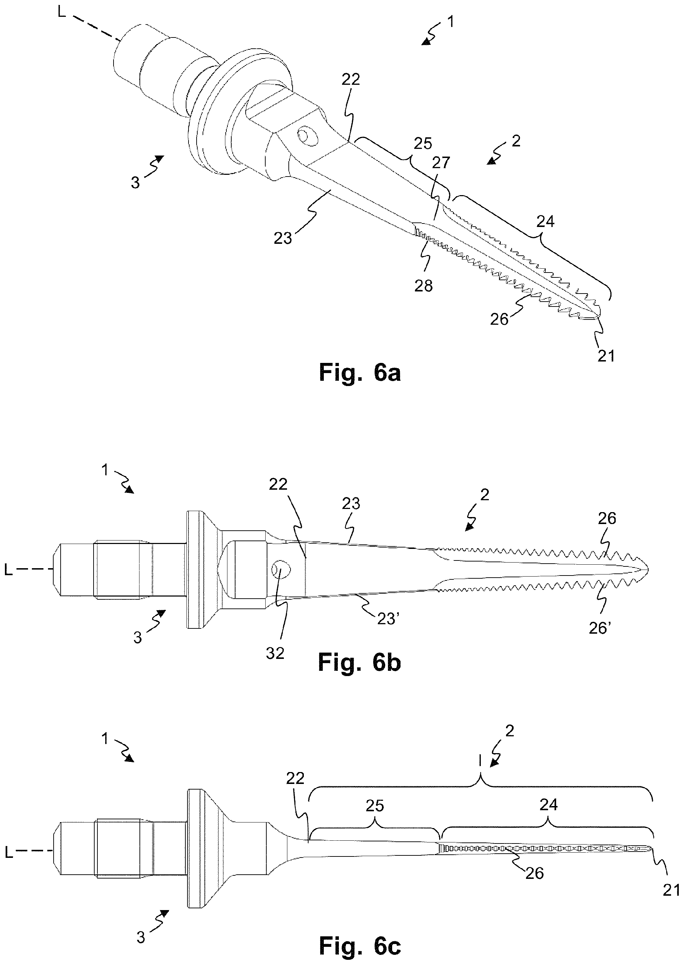

Figure 6a shows a schematic drawing of another exemplary embodiment of an ultrasonic cutting device 1 in a perspective view. The ultrasonic cutting device 1 comprises a blade member 2 with a free distal end 21, a pair of oppositely facing lateral edges, of which, in the perspective view shown in Figure 6a, only the lateral edge facing the observer is provided with the reference sign 23, and a proximal end 22 adjoining an attachment member 3 for operatively connecting the ultrasonic cutting device 1 to a source of ultrasonic oscillation. The attachment member 3 can, for example, comprise a threaded connection which can be screwed into a corresponding counter-tread in a handpiece for the ultrasonic cutting device 1. The blade member 2 comprises a distal cutting portion 24 adjoining the distal end 21 in a proximal direction, and a proximal root portion 25 adjoining the cutting portion 24 in the proximal direction. The blade member 2 and the attachment member 3 extend along a common longitudinal axis L, which is indicated by the dashed line in Figure 6a. The cutting portion 24 comprises a plurality of cutting teeth 26 that become progressively larger in a distal direction, i.e. towards the distal end 21, over substantially an entire axial length of the cutting portion 24. Put differently, the cutting portion 24 is defined along the longitudinal axis L by the presence of cutting teeth 26, with the cutting teeth 26 progressively decreasing in size from the distal end 21 toward the proximal end 22. In the embodiment shown in Figure 6a, a cross-section of the blade member 2 progressively tapers toward the distal end, with the blade member 2 in the root portion 25 having a substantially rectangular cross-section. Both the top surface 27 and the bottom surface 28 of the blade member 2 comprise two chamfers each, wherein the chamfers arranged on the top surface 27 do not adjoin on one another on the top surface 27, and wherein the chamfers arranged on the bottom surface 28 do not adjoin on one another on the bottom surface 28 of the blade member 2. Thus, a cross-section at the boundary between two neighboring cutting teeth 26 has a substantially octagonal cross-section. Accordingly, in the embodiment of the ultrasonic cutting device 1 shown in Figures 6a-6c, each tooth 26 of the plurality of cutting teeth 26 has two facets with a generally triangular shape. Starting from the distal end 21, the cutting portion 24 has cross-sections running through the tips of the cutting teeth 26, which change from a hexagonal cross-section to an octagonal cross-section in the direction of the proximal end 22.

Figure 6b shows the ultrasonic cutting device 1 of Figure 6a in a top view. As can be seen from this view, the cutting teeth 26, 26' of blade member 2 are oriented generally transversely to the longitudinal axis L. Moreover, the cutting teeth 26, 26' are arranged symmetrically in relation to the longitudinal axis L so that the teeth 26, 26' arranged on oppositely facing lateral edges 23, 23' each point in opposite directions in pairs. The distal end 21 is sharpened.

Figure 6c shows the ultrasonic cutting device 1 of Figure 6a in a side view. The blade member 2 has a working length l of about 30 mm, measured along the longitudinal axis between the distal end 21 and the proximal end 22. In other words, the distal end 21 and the proximal end 22 define the working length l along the longitudinal axis L. Accordingly, the working length l is the sum of the axial lengths of the root portion 25 and the cutting portion 24. As can be seen from both Figure 6b and Figure 6c, the cutting teeth 26 become progressively smaller in size in the proximal direction of the ultrasonic cutting tool 1 and cease to be present in the root portion 25, i.e. the root portion 25 is free of cutting teeth 26. In the embodiment shown in Figures 6a-6c, the cutting teeth 26 are arranged over about 61% of the working length 1.

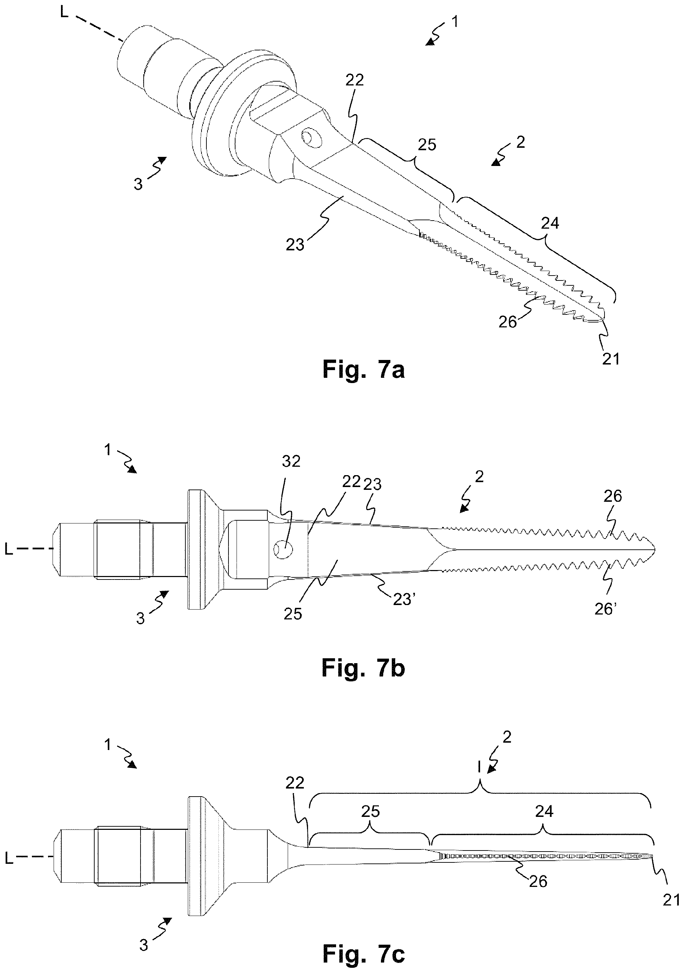

Figure 7a shows a schematic drawing of another exemplary embodiment of an ultrasonic cutting device 1 in a perspective view. The ultrasonic cutting device 1 comprises a blade member 2 with a free distal end 21, a pair of oppositely facing lateral edges, of which, in the perspective view shown in Figure 7a, only the lateral edge facing the observer is provided with the reference sign 23, and a proximal end 22 adjoining an attachment member 3 for operatively connecting the ultrasonic cutting device 1 to a source of ultrasonic oscillation. The attachment member 3 can, for example, comprise a threaded connection which can be screwed into a corresponding counter-tread in a handpiece for the ultrasonic cutting device 1. The blade member 2 comprises a distal cutting portion 24 adjoining the distal end 21 in a proximal direction, and a proximal root portion 25 adjoining the cutting portion 24 in the proximal direction. The blade member 2 and the attachment member 3 extend along a common longitudinal axis L, which is indicated by the dashed line in Figure 7a. The cutting portion 24 comprises a plurality of cutting teeth 26 that become progressively larger in a distal direction, i.e. towards the distal end 21, over substantially an entire axial length of the cutting portion 24. Put differently, the cutting portion 24 is defined along the longitudinal axis L by the presence of cutting teeth 26, with the cutting teeth 26 progressively decreasing in size from the distal end 21 toward the proximal end 22. In the embodiment shown in Figure 7a, a cross-section of the blade member 2 progressively tapers toward the distal end, with the blade member 2 in the root portion 25 having a substantially rectangular cross-section. The cutting portion 24 has four chamfers, i.e. four beveled edges, so that a cross-section at the boundary between two neighboring cutting teeth 26 has a substantially hexagonal cross-section. Accordingly, in the embodiment of the ultrasonic cutting device 1 shown in Figures 7a-7c, each tooth 26 of the plurality of cutting teeth 26 has two facets with a generally triangular shape. Starting from the distal end 21, the cutting portion 24 has cross-sections running through the tips of the cutting teeth 26, which change from a substantially diamond-shaped cross-section to a hexagonal cross-section in the direction of the proximal end 22.

Figure 7b shows the ultrasonic cutting device 1 of Figure 7a in a top view. As can be seen from this view, the cutting teeth 26, 26' of blade member 2 are oriented generally transversely to the longitudinal axis L. Moreover, the cutting teeth 26, 26' are arranged symmetrically in relation to the longitudinal axis L so that the teeth 26, 26' arranged on oppositely facing lateral edges 23, 23' each point in opposite directions in pairs. As can be seen from Figure 7b, the cutting teeth 26 become progressively smaller in size in the proximal direction of the ultrasonic cutting tool 1 and cease to be present in the root portion 25, i.e. the root portion 25 is free of cutting teeth 26.

Figure 7c shows the ultrasonic cutting device 1 of Figure 7a in a side view. The blade member 2 has a working length l of about 30 mm, measured along the longitudinal axis between the distal end 21 and the proximal end 22. In other words, the distal end 21 and the proximal end 22 define the working length l along the longitudinal axis L. Accordingly, the working length l is the sum of the axial lengths of the root portion 25 and the cutting portion 24. In the embodiment shown in Figures 7a-7c, the cutting teeth 26 are arranged over about 65% of the working length 1.

## Claims

1. An ultrasonic cutting device (1) comprising
- a blade member (2) and an attachment member (3) for operatively connecting the ultrasonic cutting device (1) to a source of ultrasonic oscillation,
- wherein the blade member (2) and the attachment member (3) extend along a common longitudinal axis L,
- wherein the blade member (2) comprises a distal end (21), a proximal end (22) adjoining the attachment member (3), and a pair of oppositely facing lateral edges (23, 23'),
- wherein the blade member (2) comprises a distal cutting portion (24) adjoining the distal end (21) in a proximal direction and comprising a plurality of cutting teeth (26), and a proximal root portion (25) adjoining the cutting portion (24) in the proximal direction,
- and wherein the cutting teeth (26) become larger, in particular progressively larger, in a distal direction over substantially an entire axial length of the cutting portion (24).

2. The ultrasonic cutting device (1) according to claim 1, wherein the cutting teeth (26) each have a tooth height h defined between a base (26a) and a tip (26b) of the respective tooth, and wherein the tooth height h of the cutting teeth (26) becomes larger in the distal direction over substantially the entire axial length of the cutting portion (24).

3. The ultrasonic cutting device (1) according to claim 2, wherein the cutting teeth (26) each have a tooth width w defined at their widest point, measured in a direction of the longitudinal axis L, and wherein the tooth width w of the plurality of cutting teeth (26) is arranged to decrease, in particular gradually, in a direction away from the distal end (21) over substantially the entire axial length of the cutting portion (24).

4. The ultrasonic cutting device (1) according to claim 2 or 3, wherein the lateral edges (23, 23') each run through the tips (26b) of the cutting teeth (26) arranged on the respective lateral edge (23, 23').

5. The ultrasonic cutting device (1) according to any one of the preceding claims, wherein the distal end (21) and the proximal end (22) define a working length l along the longitudinal axis L, the working length l being between 15 mm and 30 mm, preferably between 20 mm and 25 mm, in particular about 24 mm.

6. The ultrasonic cutting device (1) according to claim 5, wherein the cutting teeth (6) are arranged over 50% to 80% of the working length 1, preferably over 70% to 80% of the working length 1.

7. The ultrasonic cutting device () according to any one of the preceding claims, wherein the oppositely facing lateral edges (23, 23') are parallel to one another over at least 80% of the cutting portion (24), preferably over at least 90% of the cutting portion (24).

8. The ultrasonic cutting device (1) according to any one of the preceding claims, wherein the lateral edges (23, 23') in the root portion (25) are free of cutting teeth.

9. The ultrasonic cutting device (1) according to any one of the preceding claims, wherein the root portion (25) is hollow.

10. The ultrasonic cutting device (1) according to any one of the preceding claims, wherein the blade member (2), in particular its cutting portion (24), has a plurality of chamfers, in particular four chamfers.

11. The ultrasonic cutting device (1) according to any one of the preceding claims, wherein the attachment member (3) comprises a central channel (31) and at least one, preferably two irrigation apertures (32) in fluid communication with the central channel (31), wherein the at least one irrigation aperture (32) is arranged adjacent the proximal end (22) of the blade member (2) and configured for dispensing an irrigation fluid onto the blade member (2), in particular onto its root portion (25).

12. The ultrasonic cutting device (1) according to any one of the preceding claims, wherein a thickness d of the blade member (2) tapers continuously from the proximal end (22) towards the distal end (21), wherein the thickness d of the blade member (2) is defined by a distance between a top surface (27) of the blade member and a bottom surface (28) of the blade member opposite the top surface (27), measured perpendicular to a plane E extending through the plurality of cutting teeth (26).

13. The ultrasonic cutting device (1) according to claim 12, wherein the blade member (2) has a distal thickness d₂₁ of between 0.5 mm and 0.7 mm at its distal end (21) and a proximal thickness d₂₂ of at most 3 mm at its proximal end (22).

14. The ultrasonic cutting device (1) according to any one of claims 2 to 13, wherein the oppositely facing lateral edges (23, 23') define a width b of the blade member (2) transverse to the longitudinal axis L, the blade member width b being between 2 mm and 4 mm, preferably between 2.5 mm and 3.5 mm, in particular about 3 mm.

15. The ultrasonic cutting device (1) according to claim 14, wherein a sum of the tooth heights h of cutting teeth (26, 26') arranged opposite one another in the region of the distal end (21), relative to the blade member width b, is at least 40%, preferably at least 45%, in particular about 50%.
